# EUROPEAN PATENT SPECIFICATION

(11) **EP 4 202 031 B1**
(45) Date of publication and mention of the grant of the patent: **11.02.2026**
(21) Application number: 21217393.4
(22) Date of filing: 23.12.2021
(51) Int. Cl.: C12M 1/12, C12M 1/00, C12M 1/26, C12M 1/34, A61L 27/36, A61L 27/38, C12M 3/00

(54) **CELL AND TISSUE SHEET FORMING PACKAGE AND CELL INJECTION EQUIPMENT**
PAKET ZUR HERSTELLUNG VON ZELL- UND GEWEBESCHICHTEN UND AUSRÜSTUNG ZUR INJEKTION VON ZELLEN
EMBALLAGE POUR LA FORMATION DES FEUILLES DE CELLULES ET DE TISSUS ET ÉQUIPEMENT D'INJECTION DE CELLULES

(43) Date of publication of application: 28.06.2023
(73) Proprietor: Industrial Technology Research Institute, 310401 Hsinchu (TW)
(72) Inventor: HSU, HSIN-YI, 320004 Taoyuan City (TW); LIN, YANG-CHENG, 600055 Chiayi City (TW); HSU, CHAO-HONG, 710007 Tainan City (TW); LIOU, YU-BING, 300040 Hsinchu City (TW); LIN, LI-HSIN, 302050 Zhubei City (TW); SHEN, HSIN-HSIN, 310021 Zhudong Township (TW); WANG, YU-CHI, 221006 New Taipei City (TW); LI, CHANG-CHOU, 701 Tainan City (TW); HUANG, CHIH-HUNG, 730010 Tainan City (TW)
(74) Representative: Lermer, Christoph

(56) References cited:
- WO-A1-98/24880
- CN-U- 214 029 979
- JP-A- 2001 299 326
- US-A1- 2006 153 815

## Description

### TECHNICAL FIELD

The present disclosure relates in general to a cell and tissue sheet forming package and cell injection equipment.

### BACKGROUND

Cell and tissue sheets (or dressings for short) are commonly used in the medical field. By covering a wound with the cell and tissue sheet, healing speed of the wound would be increased.

Currently, a typical process for producing the cell and tissue sheets is roughly as follows. Firstly, cell collection is carried out. Then, a cell culture is performed, and further the cultured cells would be transported and stored for a subsequent surgery. However, in this process, the cell culture is extremely time-consuming and expensive because specific procedures and facilities in the laboratory are needed. As for the transportation, if the temperature, humidity and other environmental conditions are not properly controlled, then a risk of damage or contamination would be inevitable.

Accordingly, how to develop a "cell and tissue sheet forming package and associated cell injection equipment" that can simplify a cell therapy operation process, need no laboratory for culturing cells and cell membranes, waive the risk in transportation, and produce the cell sheet in situ through packaging and injection, is definitely urgent problem to be solved by those skill in the art.

Document CN 214029979 U discloses an immunochromatographic detection device having a box boy, a sliding cover 1 an iron sheet placed on the box body, and an iron sheet placed on the cover for closing and sealing the box body and the cover.

Document JP 2001 299326 A discloses a flat incubator comprising a culture cassette with a bottom portion and a sliding lid portion for culturing a cell in presence of a sheet-like support body in a liquid medium.

### SUMMARY

The present invention is directed to a cell and tissue sheet forming package comprising:
a container body, having a cavity;
a membrane, disposed in the cavity, made of a hydrophilic material; and
a sliding door plate, disposed slidably at a top of the container body in a manner of being parallel to a horizontal level surface and a first direction, the sliding door plate being slidable between a first position and a second position at the top; wherein, when the sliding door plate is at the first position, the sliding door plate covers the cavity and the membrane; wherein, when the sliding door plate is at the second position, the cavity and the membrane are exposed; the sliding door plate including:
   a hole, penetrating from a top surface of the sliding door plate to a bottom surface of the sliding door plate; wherein, when the sliding door plate, is at the first position, the hole is positioned above the cavity and the membrane for a solution disposed in the cavity, wherein said solution is a mixture of a biocompatible polymer material and cells, to be injected therethrough into the cavity from the top surface of the sliding door plate; and
   a passive magnetic assembly, disposed at the sliding door plate for connecting magnetically a positive magnetic assembly, the positive magnetic assembly being configured to slide the sliding door plate; and
   a sealing film, configured to cover the sliding door plate and the container body so as to seal the sliding door plate and the membrane.

A further aspect of the present invention is a cell injection equipment comprising the aforementioned cell and tissue sheet forming package, comprising:
a carrier, including:
a base block, configured to carry the cell and tissue sheet forming package;
a top block, disposed pivotally on the base block for positioning the cell and tissue sheet forming package inside the base block, further having an injection hole; wherein, when the top block is closed on the base block, a projection area of the injection hole and another projection area of the hole of the sliding door plate of the cell and tissue sheet forming package are overlapped;
at least one heating element, disposed at the base block for providing thermal energy to the membrane and the solution; and
a positive magnetic assembly, disposed slidably between a third position and a fourth position at a top of the top block in a manner of being parallel to the first direction, configured to magnetically connect the passive magnetic assembly; wherein, when the positive magnetic assembly slides to the fourth position, the sliding door plate is slided synchronously to the second position;
an injection mechanism, including:
   a fixing set, mounted on a rack extending in perpendicular to the horizontal level surface;
   an injection assembly, including:
      a tube body, configured to contain the solution, disposed at the fixing set by being perpendicular to the horizontal level surface;
      an injection head, coaxially disposed to a bottom of the tube body, having an injection tip protruding toward the horizontal level surface; and
      a piston member, coaxially disposed in the tube body; and
      a drive mechanism, including:
         a first drive assembly, configured to drive the carrier to undergo a reciprocating movement parallel to the horizontal level surface and the first direction between a fifth position and a sixth position; wherein, when the carrier is at the fifth position, a projection area of the injection head is deviated from another projection area of the injection hole of the top block; wherein, when the carrier is at the sixth position, the projection area of the injection head, the another projection area of the injection hole of the top block, and a further projection area of the hole of the sliding door plate are overlapped;
         a second drive assembly, configured to drive the fixing set and the injection assembly to undergo another reciprocating movement along the rack perpendicular to the horizontal level surface between a seventh position and an eighth position; wherein, when the fixing set and the injection assembly are at the seventh position, a horizontal position of the injection tip is higher than that of a top surface of the top block of the carrier; wherein, when the fixing set and the injection assembly are at the eighth position, the horizontal position of the injection tip is lower than that of the sealing film on the hole of the sliding door plate; and
         a third drive assembly, configured to drive the piston member to move perpendicular to the horizontal level surface inside the tube body for pushing the solution out of the tube body via the injection head.

### BRIEF DESCRIPTION OF THE DRAWINGS

FIG. 1 is a schematic perspective view of an embodiment of the cell and tissue sheet forming package in accordance with this disclosure;
FIG. 2 is a schematic exploded view of FIG. 1 with the sealing film removed;
FIG. 3 is a schematic perspective view of FIG. 1 with the sealing film removed and the sliding door plate to be at the second position;
FIG. 3A is a schematic cross-sectional view of FIG. 3 along line 3A-3A;
FIG. 4 is a schematic perspective view of FIG. 1 with the sealing film removed and the sliding door plate to be at the first position;
FIG. 4A is a schematic cross-sectional view of FIG. 4 along line 4A-4A;
FIG. 5 and FIG. 5A are two schematic perspective view of FIG. 1 having a constraint frame, with the constraint frame separated and engaged, respectively;
FIG. 6 is a schematic perspective view of an embodiment of the cell injection equipment in accordance with this disclosure;
FIG. 7 is a schematic perspective view of an embodiment of the carrier for containing therein the cell and tissue sheet forming package of FIG. 1, with the top block of the carrier opened from the base block thereof;
FIG. 8 is a schematic cross-sectional view showing that the carrier is at a fifth position and a projection area of the injection head is deviated from a projection area of the injection hole of the top block in accordance with this disclosure;
FIG. 8A is a schematic cross-sectional view showing that the carrier is at a sixth position and a projection area of the injection head 622 is coincided with a projection area of the injection hole of the top block in accordance with this disclosure;
FIG. 9 demonstrates schematically a state that the injection assembly of the injection device is lowered to an eighth position in accordance with this disclosure;
FIG. 9A is a schematic cross-sectional view showing that a horizontal position of the injection tip of the injection device is lower than a horizontal position of the bottom surface of the sliding door plate in accordance with this disclosure;
FIG. 10 demonstrates schematically a state that the injection assembly of the injection device is raised to a seventh position and the heating element is heating in accordance with this disclosure;
FIG. 11 demonstrates schematically a state that the positive magnetic assembly of the injection device is slided to a fourth position in accordance with this disclosure;
FIG. 12 demonstrates schematically a state that the top block of the injection device is opened and the cell and the tissue sheet forming package is ready to be fetched in accordance with this disclosure;
FIG. 13 demonstrates schematically a state that the cell and tissue sheet is removing from the cell and tissue sheet forming package in accordance with this disclosure; and
FIG. 14 demonstrates schematically a state that the cell and tissue sheet is removed from the cell and tissue sheet forming package furnished with the constraint frame in accordance with this disclosure.

### DETAILED DESCRIPTION

In this disclosure, with the cell and tissue sheet forming package and the cell injection equipment, since the cell sheets are produced on-site in the clinic, thus the cell treatment procedures can be simplified, no pre-work is required in the laboratory to culture the cells and further to form the cell sheets, and so the risk in temperature control during transportation can be effectively avoided.

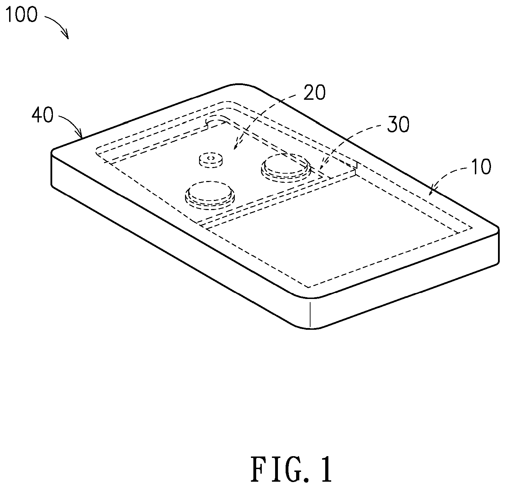

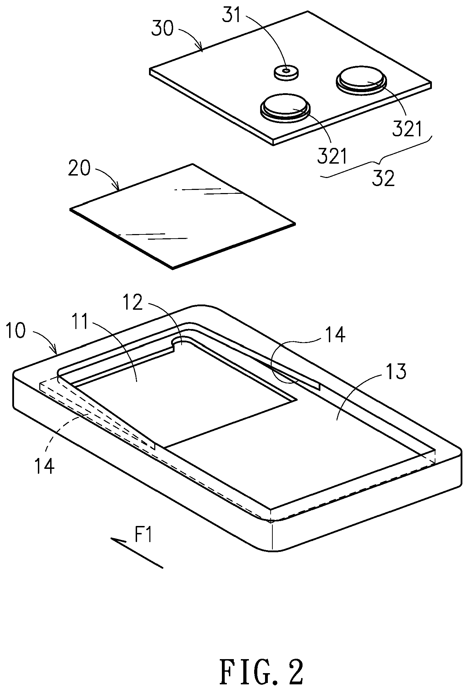

Referring to FIG. 1 and FIG. 2, a cell and tissue sheet forming package 100 includes a container body 10, a membrane 20, a sliding door plate 30 and a sealing film 40.

The container body 10 and the sliding door plate 30 can be made of one of PET, PS, PP, PVC, PE, PC, ABS and PTFE.

The membrane 20, as a hydrophilic material, can be made of one of PLA, PCL and collagen.

The sealing film 40 can be made of one of Tyvek, Al foil and nylon.

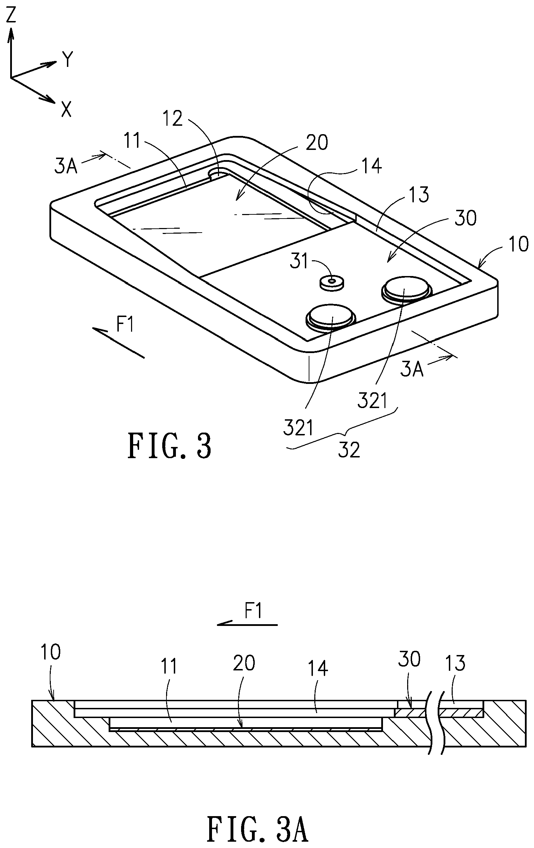

Referring to FIG. 2, FIG. 3 and FIG. 3A, the container body 10 has a cavity 11 having a protrusive corner 12 at a side thereof, and the protrusive corner 12 is spatially connected with the cavity 11.

The membrane 20 is to be placed into the cavity 11. After the membrane 20 is placed into the cavity 11, an edge of the membrane 20 would be exposed at the protrusive corner 12, such that a gap would exist between the membrane 20 and an inner wall of the protrusive corner 12. With this gap, the membrane 20 can be easily fetched and thus removed from the cavity 11.

The container body 10 has a shallow area 13, and the cavity 11 is disposed in this shallow area 13. At two inner opposite sides of the container body 10 in the shallow area 13 with respect to the cavity 11, a pair of slideways 14 are furnished respectively to extend parallel to a first direction F1.

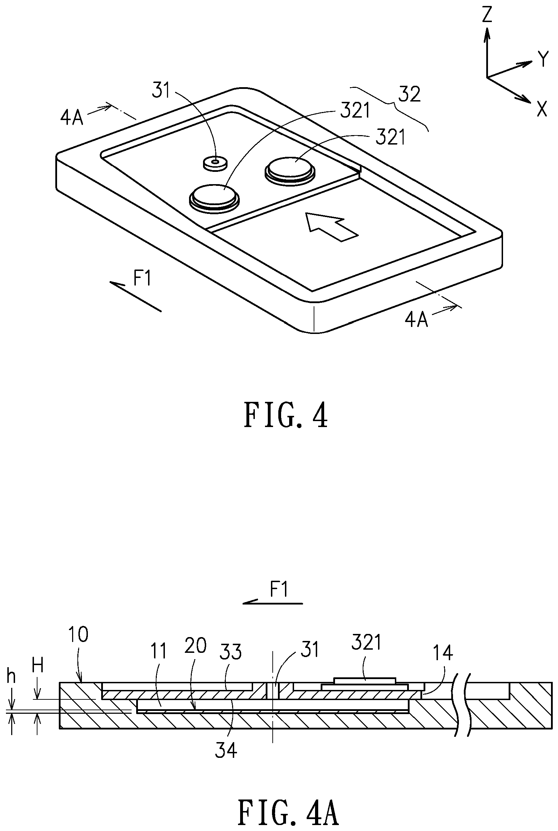

Referring to FIG. 3, FIG. 3A, FIG. 4 and FIG. 4A, the sliding door plate 30 is provided to slide along the two slideways 14. The sliding door plate 30, parallel to a horizontal level surface as well as the first direction F1, can slide in the first direction F1 between a first position and a second position at an upper portion of the container body 10. Hereinafter, the horizontal level surface would be specifically directed to an X-Y plane spanned by the X and Y axises as shown.

When the sliding door plate 30 is at the second position, the cavity 11 and the membrane 20 can be exposed, as shown in FIG. 3. When the sliding door plate 30 is at the first position, the cavity 11 and the membrane 20 would be covered, as shown in FIG. 4.

Referring to FIG. 3, FIG. 3A, FIG. 4 and FIG. 4A, the sliding door plate 30 includes a hole 31 and a passive magnetic assembly 32.

The hole 31 is configured to penetrate through from a top surface 33 of the sliding door plate 30 to a bottom surface 34 of the sliding door plate 30. When the sliding door plate 30 is at the first position shown in FIG. 4, the hole 31 is located above the cavity 11 and the membrane 20. The hole 31 is configured to allow a solution (not shown in the figure) to enter the cavity 11 from the top surface 33 of the sliding door plate 30. Referring to FIG. 4A, in this embodiment, when the sliding door plate 30 is at the first position, a projection area of the hole 31 is right at a center of the cavity 11 or the membrane 20, such that the introduced solution can be distributed much more uniformly.

In this disclosure, the solution is a mixture of a biocompatible polymer material and cells, in which the polymer material can be one of collagen, gelatin, hyaluronic acid, alginate, PEG and any copolymer the like, and the cells can be one of fibroblasts, myoblasts, epithelial cells, endothelial cells, precursor cells, tenocytes, stem cells, mesenchymal stem cells, bone marrow stem cells and adipose stem cells.

The passive magnetic assembly 32 is adopted to connect magnetically a positive magnetic assembly (not shown in the figure). Driven by the positive magnetic assembly, the sliding door plate 30 having the passive magnetic assembly 32 can slide between the first position and the second position in the first direction F1 and parallel to the horizontal level surface. In this embodiment, the passive magnetic assembly 32 has two passive magnetic elements 321. These two passive magnetic elements 321 are disposed on the same horizontal level surface, and a connection line of these to passive magnetic elements 321 is perpendicular to the first direction F1.

Referring to FIG. 4A, the cavity 11 has a depth H parallel to a Z-axis direction (i.e., perpendicular to the horizontal level surface), and the membrane 20 also has a thickness h parallel to the Z-axis direction and perpendicular to the horizontal level surface. The depth H is larger than the thickness h. The cavity 11 has an area A on the horizontal level surface (i.e., the bottom surface area). Thus, the maximum volume of the solution able to be provided to the cavity 11 is about (H-h)×A. Generally, the thickness h of the membrane 20 is within 10~100µm. For example, if the membrane 20 has a size of 30×30×0.02mm, then the solution required is about 0.9~1.1ml. According to the (H-h)×A, the reference depth H of the cavity 11 can be derived.

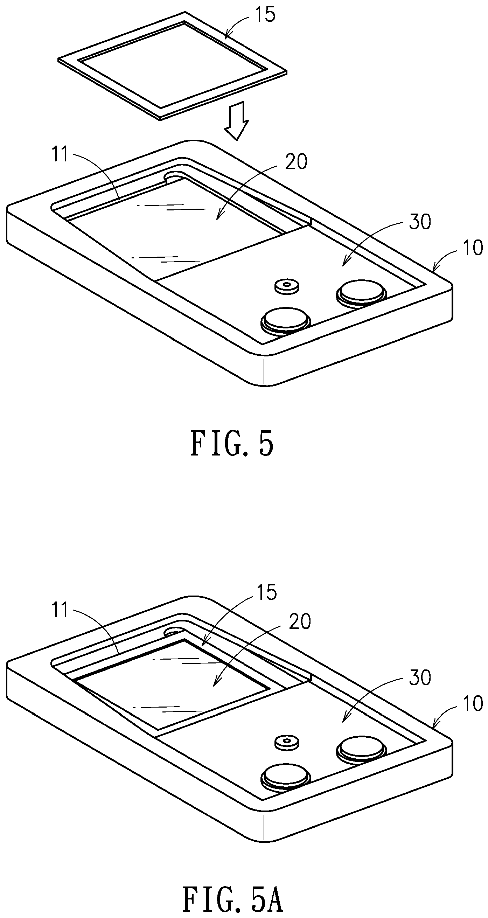

Referring to FIG. 5 and FIG. 5A, in this embodiment, the cavity 11 is furnished with a constraint frame 15. The constraint frame 15 is introduced to tightly fit the cavity 11 so as to fixedly position the membrane 20 thereunder in the cavity 11. With the constraint frame 15 to depress at a circumferential rim of a top surface of the membrane 20, then the membrane 20 can be disposed in the cavity 11 fixedly and stably.

In this disclosure, the constraint frame 15 can be made of one of PET, PS, PP, PVC, PE, PC, ABS, PTFE, rubber and silicon.

Referring to FIG. 1, FIG. 3 and FIG. 4, the membrane 20 and sliding door plate 30 are both disposed in the container body 10 firstly as shown in FIG. 3, then the sliding door plate 30 is moved to the first position for covering the membrane 20 as shown in FIG. 4, and finally the sealing film 40 is applied to cover the sliding door plate 30 and an upper surface of the container body 10 so as to seal the sliding door plate 30 and the membrane 20 inside the sealing film 40. Thereupon, the cell and tissue sheet forming package 100 of FIG. 1 can be formed.

Similarly, in the embodiment of FIG. 5 and FIG. 5A, the sliding door plate 30 and the upper surface of the container body 10 can be sealed by the sealing film 40 in accordance to the arrangement shown in FIG. 3 and FIG. 4. Thus, a package similar to the cell and tissue sheet forming package 100 of FIG. 1 can be obtained.

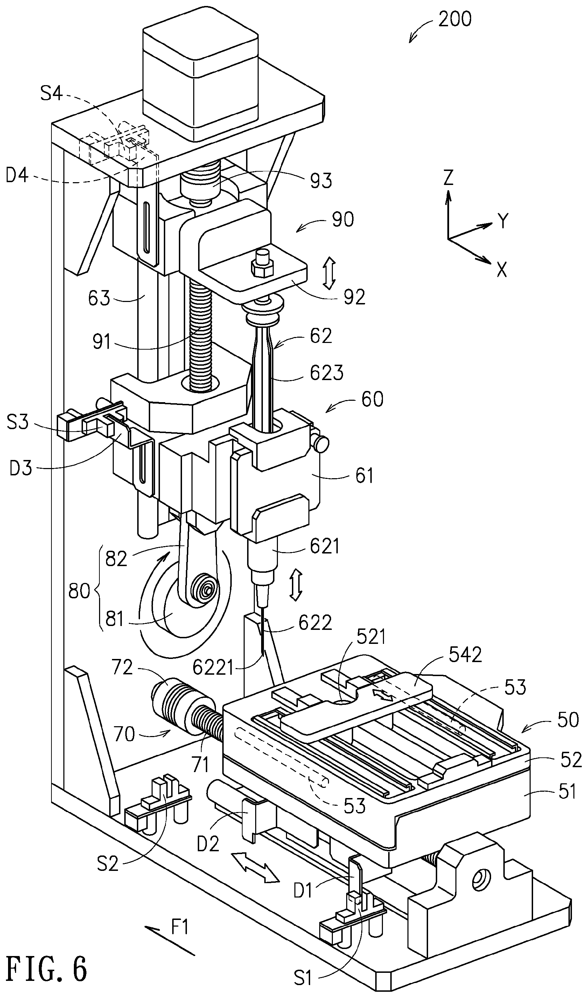

Referring to FIG. 6, a cell injection equipment 200 is provided to perform the injection of the solution to the cell and tissue sheet forming package 100 of FIG. 1.

The cell injection equipment 200 includes a carrier 50 and an injection mechanism 60. In addition, a drive mechanism consisted of at least a first drive assembly 70, a second drive assembly 80 and a third drive assembly 90 is configured to drive the carrier 50 and the injection mechanism 60.

Referring to FIG. 6, FIG. 7 and FIG. 8, the carrier 50 includes a base block 51, a top block 52, two heating elements 53 and a positive magnetic assembly 54.

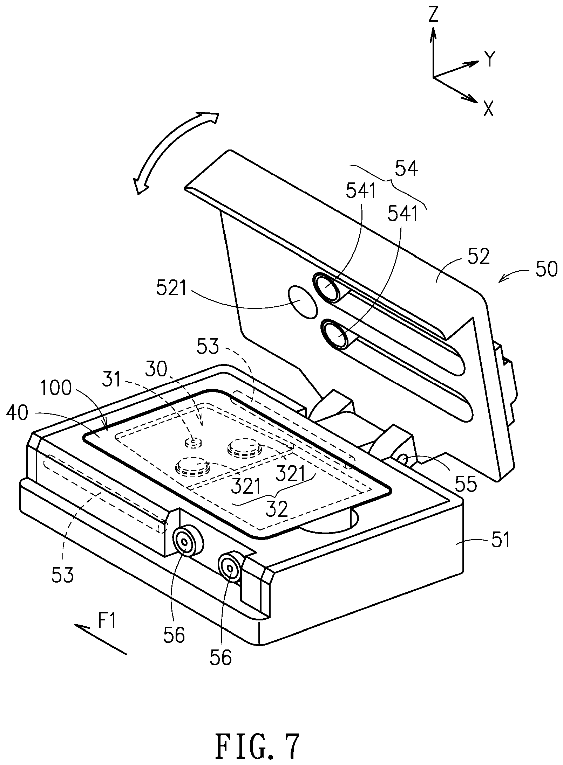

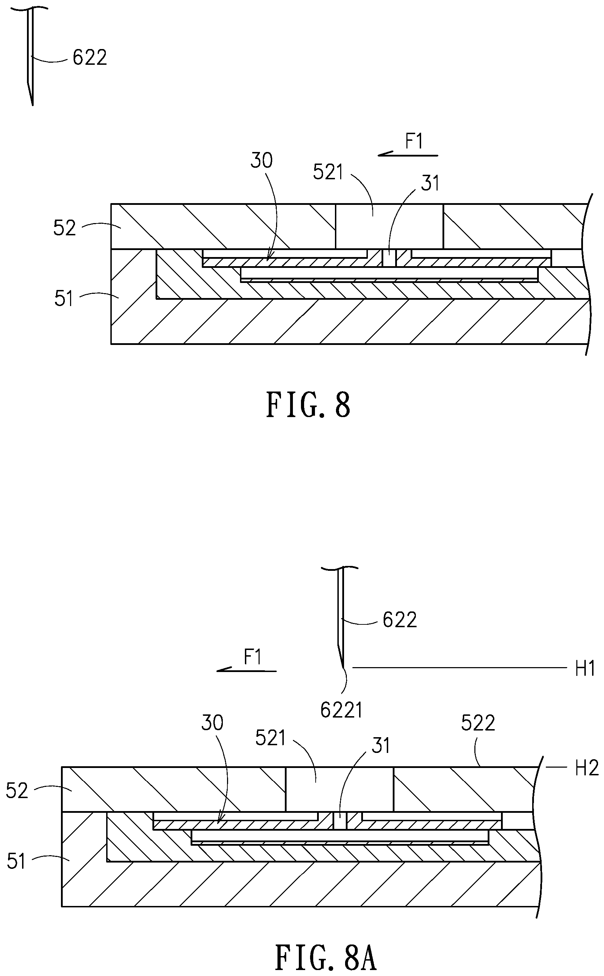

The base block 51 is relevant to carry the cell and tissue sheet forming package 100 of FIG. 1. The top block 52 is connected with the base block 51. In this embodiment, the top block 52 is connected pivotally with the base block 51 via a hinge 55 at one side thereof. While in an opposite side, at least one magnet member 56 is applied to connect the top block 52 to the base block 51 in a detachable manner, such that the top block 52 can be opened from or closed onto the base block 51. When the top block 52 is closed onto the base block 51, the cell and tissue sheet forming package 100 can be fixedly positioned inside the base block 51. Since the sliding door plate 30 and the other members of the cell and tissue sheet forming package 100 are sealed by the sealing film 40, thus dashed lines thereto are used in FIG. 7. The top block 52 has an injection hole 521. When the top block 52 closes the base block 51, a projection area of the injection hole 521 is overlapping a projection area of the hole 31 of the sliding door plate 30 of the cell and tissue sheet forming package 100, as shown in FIG. 8.

The heating elements 53 are disposed at the base block 51. In this embodiment, two heating elements 53 are shown to two opposite lateral sides of the base block 51, for heating the membrane 20 and the solution of the cell and tissue sheet forming package 100 in between thereof. In this embodiment, each of the heating elements 53 is configured into a tube shape as shown in FIG. 7; but, according to this disclosure, the configuration of the heating element 53 is not limited thereto.

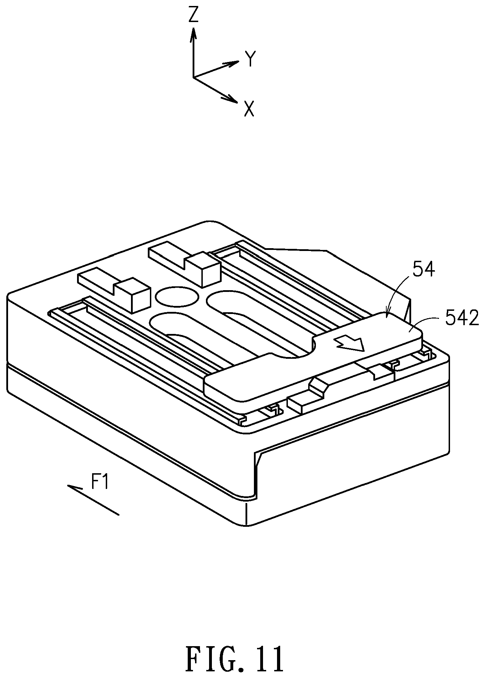

The positive magnetic assembly 54, disposed at the top of the tip block 52, is slidable in the first direction F1 between a third position and a fourth position. The positive magnetic assembly 54 includes two positive magnetic elements 541 connected with a push member 542. When the top block 52 and the base block 51 are in the close state, the two positive magnetic elements 541 of the positive magnetic assembly 54 is right to connect magnetically the two corresponding passive magnetic elements 321 of the passive magnetic assembly 32 at the cell and tissue sheet forming package 100. Thereupon, by displacing the push member 542 connected with the two positive magnetic elements 54, the positive magnetic assembly 54 can then slide between the third position and the fourth position (referring to the position of the positive magnetic assembly 54 in FIG. 11). As such, the sliding door plate 30 of the cell and tissue sheet forming package 100 can be then synchronously moved to slide between the first position and the second position (referring to the position of the sliding door plate 30 in FIG. 3).

In this embodiment, with the cell and tissue sheet forming package 100 to be disposed in the carrier 50, the magnetic connection between the positive magnetic assembly 54 and the passive magnetic assembly 32 is utilized to control the movement of the sliding door plate 30 of the cell and tissue sheet forming package 100 outside the carrier 50. In one embodiment, the push member 542 can be also manipulated manually, mechanically or electrically to move the positive magnetic assembly 54 back and forth.

Referring to FIG. 6, the injection mechanism 60 includes a fixing set 61 and an injection assembly 62. The fixing set 61 is mounted to a rack 63 that is extended in parallel to the Z-axis direction but perpendicular to the horizontal level surface.

The injection assembly 62 includes a tube body 621, an injection head 622 and a piston member 623. The tube body 621, formed as a lengthy tube disposed at the fixing set 61, is configured to contain the solution. An axial of the tube body 621 is parallel to the Z-axis direction but perpendicular to the horizontal level surface. The injection head 622, coaxially disposed to a bottom of the tube body 621, has an injection tip 6221 extended toward the horizontal level surface. The piston member 623, axially disposed in the tube body 621, is slidable inside the tube body 621 in the Z-axis direction so as to push out the solution inside the tube body 621 via the injection tip 6221 of the injection head 622.

In the following description, movements of the first drive assembly 70, the second drive assembly 80 and the third drive assembly 90 of the drive mechanism will be elucidated.

Referring to FIG. 6, FIG. 8 and FIG. 8A, the first drive assembly 70 is utilized to displace the carrier 50, particularly to move in the first direction F1 by being parallel to the horizontal level surface. The first drive assembly 70 includes a first screw bar 71 and a first motor 72. An axial direction of the first screw bar 71 is parallel to the horizontal level surface, and the carrier 50 is disposed to the first screw bar 71. The first motor 72 is to rotate the first screw bar 71 clockwise and counter clockwise, and thereby to synchronously drive the carrier 50 to undergo a reciprocating movement between a fifth position and a sixth position.

A first sensor S1 and a second sensor S2 are located at a side of the carrier 50, and a first detection plate D1 and a second detection plate D2 are provided to the base block 51 of the carrier 50 at positions of the same side thereof in correspondence to the first sensor S1 and the second sensor S2, respectively. The first detection plate D1 and the second detection plate D2 are to be detected by the first sensor S1 and the second sensor S2, respectively, so as to capture position information of the carrier 50. When the first drive assembly 70 drives the carrier 50 to displace, the first detection plate D1 and the second detection plate D2 would move along with the carrier 50 synchronously. If the carrier 50 is moved, then the first detection plate D1 and the second detection plate D2 would gradually enter detection ranges of the first sensor S1 and the second sensor S1, such that the carrier 50 can be controlled to stop at the fifth position or the sixth position.

As soon as the first sensor S1 detects the first detection plate D1, then the first drive assembly 70 would be controlled to stop so as to have the carrier 50 to stop at the fifth position, as shown in FIG. 6. At this time, the projection area of the injection head 622 would be deviated from the projection area of the injection hole 521 of the top block 52, as shown in FIG. 8.

Referring to FIG. 6, when the first drive assembly 70 drives the carrier 50 to move toward one side of the injection mechanism 60 so as to have the second sensor S2 to detect the second detection plate D2, then the first drive assembly 70 is controlled to stop the carrier 50 at the sixth position. At this time the projection area of the injection head 622, that of the injection hole 521 of the top block 52, and that of the hole 31 of the sliding door plate 30 would be coincided, as shown in FIG. 8A. Then, the second drive assembly 80 can proceed to drive the fixing set 61 and the injection assembly 62 to move.

Referring to FIG. 6, the second drive assembly 80 is to drive the fixing set 61 and the injection assembly 62 to move. The second drive assembly 80 includes a cam member 81, a link member 82 and a drive member (not shown in the figure), in which the drive member can be a motor. The cam member 81 is disposed under the fixing set 61. One end of the link member 82 is pivotally connected to the fixing set 61, while another end thereof is pivotally connected to an eccentric point of the cam member 81. When the drive member rotates the cam member 81, the link member 82 would be driven synchronously to move the fixing set 61. In this embodiment, the fixing set 61 and the injection assembly 62 would undergo a reciprocating movement between a seventh position and an eighth position, parallel to the Z-axis direction along the rack 63, and perpendicular to the horizontal level surface.

A third sensor S3 is located beside the injection mechanism 60, and a third detection plate D3 to be detected by the third sensor S3 so as for obtaining the position information of the fixing set 61 and the injection assembly 62 of the injection device 60 is provided to the fixing set 61 at the same side where the third sensor S3 is located. When the second drive assembly 80 drives the fixing set 61 and the injection assembly 62 to move, the third detection plate D3 may move synchronously so as further to enter a detection range of the third sensor S3. When the third sensor S3 detects the third detection plate D3, then the second drive assembly 80 is controlled to stop so as to have the fixing set 61 stopped at the seventh position, as shown in FIG. 6. At this time, as shown in FIG. 8A, the horizontal position H1 of the injection tip 6221 is higher than the horizontal position H2 of the tip surface 522 of the top block 52 of the carrier 50.

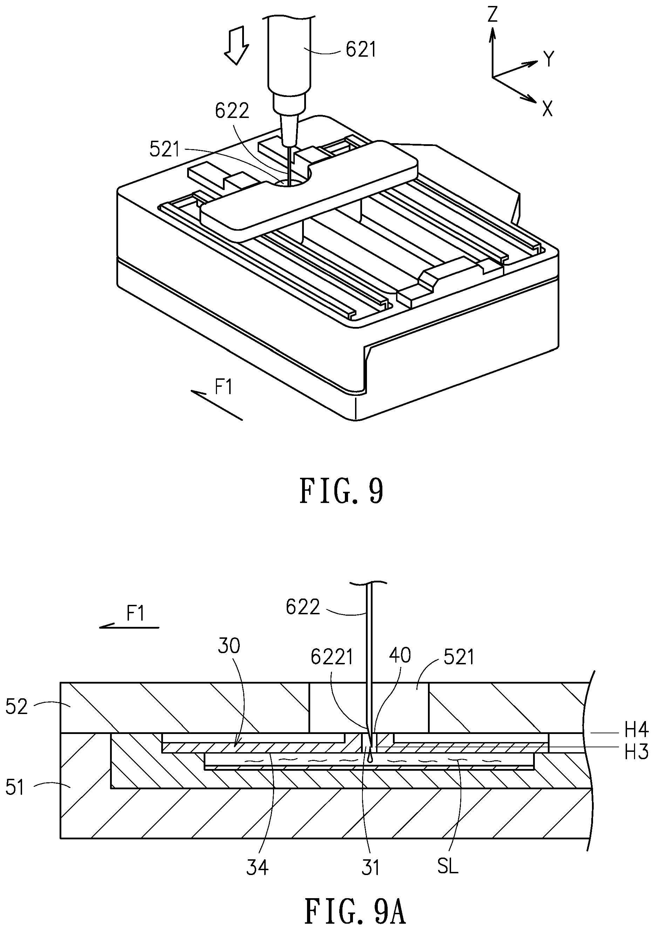

Referring to FIG. 6, FIG. 9 and FIG. 9A, when the fixing set 61 and the injection assembly 62 are driven downward to the eighth position, the injection head 622 would enter the injection hole 521. Since the horizontal position H3 of the injection tip 6221 is lower than the horizontal position H4 of the sealing film 40 at the top of the hole 31, and the projection area of the injection head 622 and that of the injection hole 521 of the top block 52 are overlapped, thus the injection tip 6221 can pierce the sealing film 40 to enter the hole 31. Then, the third drive assembly 90 can be activated to depress the piston member 623 of the injection assembly 62.

Referring to FIG. 6, the third drive assembly 90 is configured to move the piston member 623. The third drive assembly 90 includes a second screw bar 91, a depression member 92 and a second motor 93. An axial direction of the second screw bar 91 is parallel to the Z-axis direction but perpendicular to the horizontal level surface. The depression member 92, disposed to the second screw bar 91, has a lower side to face a top surface of the piston member 623, in which the lower side is used to directly depress the piston member 623. The second motor 93 can drive the second screw bar 91 to rotate clockwise and counter clockwise so as to synchronously displace the depression member 92 linearly in a reciprocating manner between a ninth position and a tenth position, by being parallel to the Z-axis direction but perpendicular to the horizontal level surface.

One side of the cell injection equipment 200 is further furnished with a fourth sensor S4, and, at the same side, the depression member 92 of the third drive assembly 90 is provided with a fourth detection plate D4 to pair the fourth sensor S4. The fourth detection plate D4 is used to be detected by the fourth sensor S4 so as to obtain the position information of the depression member 92. When the third drive assembly 90 drives the depression member 92 to move, the fourth detection plate D4 would displace synchronously to approach the detection range of the fourth sensor S4. In FIG. 6, the depression member 92 is shown to be at the ninth position where the fourth sensor S4 can detect the fourth detection plate D4, and then the third drive assembly 90 is controlled to stop. At this time, the depression member 92 does not contact the piston member 623, and the fixing set 61 is at the seventh position.

Referring to FIG. 9 and FIG. 9A, when the second drive assembly 80 drives the fixing set 61 and the injection assembly 62 to move downward to the eighth position as shown in FIG. 9 and FIG. 9A, the third drive assembly 90 would then lower the depression member 92 in the Z-axis direction but perpendicular to the horizontal level surface, such that the depression member 92 can contact the piston member 623. With the depression member 92 continues to reach the tenth position so as to push out the solution SL originally inside the tube body 621 via the injection head 622, then the solution SL would enter the space between the sliding door plate 30 and the membrane 20, as shown in FIG. 9A.

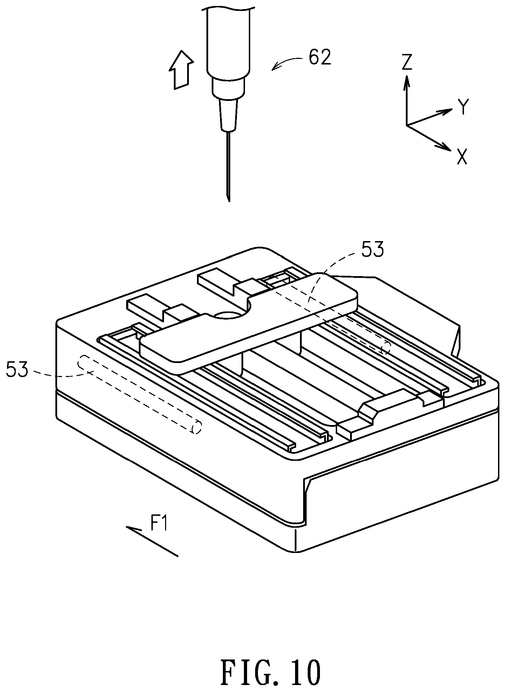

Referring to FIG. 6, FIG. 9A and FIG. 10, after the solution SL is completely injected into the space between the sliding door plate 30 and the membrane 20, the third drive assembly 90 would drive the depression member 92 upward to retrieve from the contact of the piston member 623. When the depression member 92 is raised to the ninth position as shown in FIG. 6, the fourth sensor S4 would detect the fourth detection plate D4, and the third drive assembly 90 would be controlled to stop.

Then, the second drive assembly 80 is controlled to drive the fixing set 61 and the injection assembly 62 upward to the seventh position as shown in FIG. 6. When the third sensor S3 detects the third detection plate D3, the second drive assembly 80 is controlled to stop, such that the fixing set 61 can stop at the seventh position, as shown in FIG. 6.

Then, the heating element 53 would provide thermal energy to the membrane 20 and the solution SL of the cell and tissue sheet forming package 100, such that the solution SL can be transformed into a colloid sheet 21 attached to the membrane 20. In one embodiment, to a 30×30×0.02mm membrane 20 and a 0.9~1.1ml solution SL, if the heating element 53 is heated up to 37~40°C, then about 10 minutes are enough for the solution SL to form the colloid sheet 21 attached on the membrane 20.

Then, as shown in FIG. 11, by moving the push member 542 in the first direction F1, the positive magnetic assembly 54 can be slided to the fourth position. Since the two positive magnetic elements 541 of the positive magnetic assembly 54 can be connected magnetically with the two passive magnetic elements 321 of the passive magnetic assembly 32 of the cell and tissue sheet forming package 100, thus the sliding door plate 30 can be synchronously slided to the second position (referring to FIG. 3 for the position of the sliding door plate 30). In addition, since the sliding door plate 30 is moved by sliding, thus any adhesive from the colloid sheet 21 on the membrane 20 can be avoided.

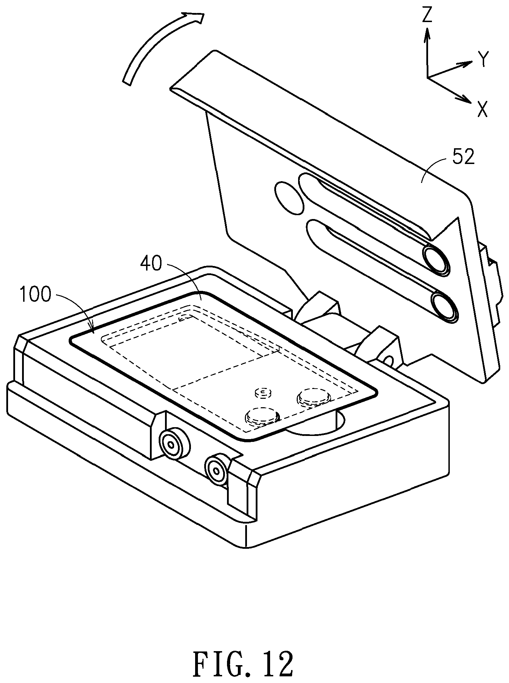

Then, as shown in FIG. 12, after the top block 52 is opened, the cell and tissue sheet forming package 100 can be fetched out from the base block 51, and thus the sealing film 40 wrapping the cell and tissue sheet forming package 100 can be peeled off.

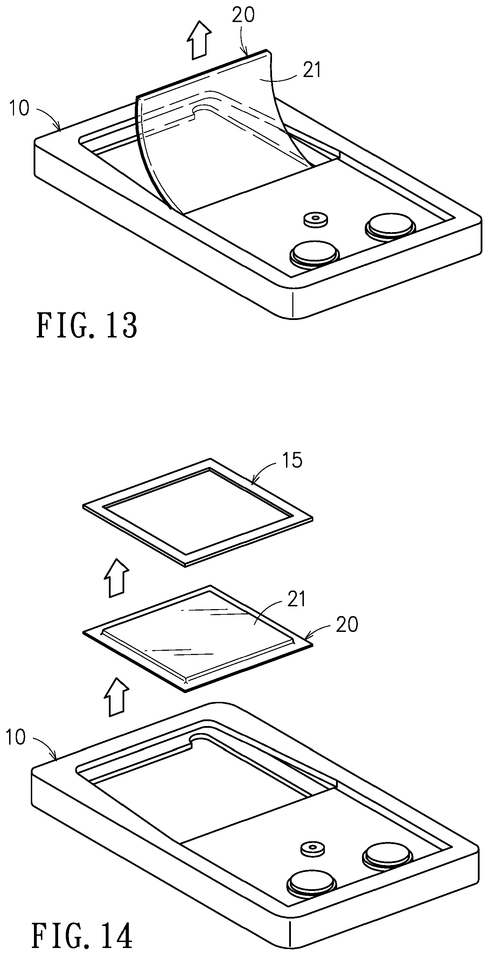

Further, as shown in FIG. 13, the membrane 20 attached thereon with the colloid sheet 21 (i.e., the cell and tissue sheet) can be removed from the container body 10 for a direct treatment on a wound.

If the constraint frame 15 of FIG. 5 and FIG. 5A is applied on the membrane 20, then, as shown in FIG. 14, both the constraint frame 15 and the membrane 20 attached thereon with the colloid sheet 21 are removed together from the container body 10. Then, the constraint frame 15 and the membrane 20 with the colloid sheet 21 are separated, and the membrane 20 with the colloid sheet 21 attached thereon (i.e., the cell and tissue sheet) can be directly applied on a wound of a patient.

In summary, by providing the cell and tissue sheet forming package and cell injection equipment of this disclosure, the membrane is pre-sealed inside the cell and tissue sheet forming package. Thus, when a cell and tissue sheet is required, the cell and tissue sheet forming package is placed into the carrier of the cell injection equipment by the on-site medical personnel. Then, the injection assembly having the solution prepared by mixing cells and the predetermined polymer material can be mounted onto the injection mechanism of the cell injection equipment. As the cell injection equipment is turned on, the carrier can be moved to the position under the injection mechanism, and then the injection assembly is lowered to inject the solution into the cell and tissue sheet forming package. After necessary heating, the magnetic assemblies can be applied to slide the sliding door plate away from the cell and tissue sheet forming package, and then the membrane having thereon the colloid sheet (i.e., the cell and tissue sheet) can be removed directly for further treatment.

As described above, empirically, to a 30×30×0.02mm membrane 20 and a 0.9~1.1ml solution SL, with the heating element 53 to heat up to 37~40°C for about 10 minutes, then the solution SL can be transformed into the colloid sheet 21 attached on the membrane 20. In accordance with this disclosure, the cell treatment procedures can be significantly simplified, no tedious operation in the laboratory for culturing the cells and forming the cell sheets is necessary, and thus the risk in temperature control while transporting the cell products can be avoided. With the pre-prepared package and the cell injection equipment, the cell sheets can be produced on-site in the clinic. By peeling the sterile package, the cell and tissue sheet thereinside can be directly applied to the patient, and thus the entire treatment can be substantially shortened and simplified.

## Claims

1. A cell and tissue sheet forming package (100), comprising:
a container body (10), having a cavity (11);
a membrane (20), disposed in the cavity (11), made of a hydrophilic material; and
a sliding door plate (30), disposed slidably at a top of the container body (10) in a manner of being parallel to a horizontal level surface and a first direction (F1), the sliding door plate (30) being slidable between a first position and a second position at the top; wherein, when the sliding door plate (30) is at the first position, the sliding door plate (30) covers the cavity (11) and the membrane (20); wherein, when the sliding door plate (30) is at the second position, the cavity (11) and the membrane (20) are exposed;
**characterized in that** the sliding door plate (30) includes:
a hole (31), penetrating from a top surface (33) of the sliding door plate (30) to a bottom surface (34) of the sliding door plate (30); wherein, when the sliding door plate (30) is at the first position, the hole (31) is positioned above the cavity (11) and the membrane (20) for a solution (SL) disposed in the cavity, wherein the solution (SL) is a mixture of a biocompatible polymer material and cells, to be injected therethrough into the cavity (11) from the top surface (33) of the sliding door plate (30); and
a passive magnetic assembly (32), disposed at the sliding door plate (30) for connecting magnetically a positive magnetic assembly (54), the positive magnetic assembly (54) being configured to slide the sliding door plate (30); and
a sealing film (40), configured to cover the sliding door plate (30) and the container body (10) so as to seal the sliding door plate (30) and the membrane (20).

2. The cell and tissue sheet forming package (100) of claim 1, further including a constraint frame (15) configured to fit tightly the cavity (11) and thus able to depress on a circumferential rim of the membrane (20).

3. The cell and tissue sheet forming package (100) of claim 1, wherein a depth of the cavity (11) is greater than a thickness of the membrane (20).

4. The cell and tissue sheet forming package (100) of claim 1, wherein the cavity (11) has a depth H perpendicular to the horizontal level surface, the membrane (20) has a thickness h perpendicular to the horizontal level surface, the cavity (11) has an area A parallel to the horizontal level surface, and a maximum volume of the solution (SL) is (H-h)×A.

5. The cell and tissue sheet forming package (100) of claim 1, wherein the container body (10) has a shallow area (13), the cavity (11) is disposed beneath the shallow area (13), two slideways (14) are furnished to opposite sides of the shallow area (13) in correspondence to the cavity (11), the two slideways (14) are individually extended in the first direction (F1), and the sliding door plate (30) is disposed between the two slideways (14).

6. The cell and tissue sheet forming package (100) of claim 1, wherein the cavity (11) further has a protrusive corner (12), the protrusive corner (12) and the cavity (11) are connected spatially, and an edge of the membrane (20) is exposed to the protrusive corner (12) so as to produce a gap between the membrane (20) and the protrusive corner (12).

7. The cell and tissue sheet forming package (100) of claim 1, wherein, when the sliding door plate (30) is at the first position, a projection area of the hole (31) is coincided with centers of the cavity (11) and the membrane (20).

8. The cell and tissue sheet forming package (100) of claim 1, wherein the passive magnetic assembly (32) has two passive magnetic elements (321) parallel to the horizontal level surface, and a line connecting the two passive magnetic elements (321) is perpendicular to the first direction (F1).

9. The cell and tissue sheet forming package (100) of claim 1, wherein the polymer material is one of collagen, gelatin, hyaluronic acid, alginate, and PEG.

10. The cell and tissue sheet forming package (100) of claim 9, wherein the cells are one of fibroblasts, myoblasts, epithelial cells, endothelial cells, precursor cells, tenocytes, stem cells, mesenchymal stem cells, bone marrow stem cells and adipose stem cells.

11. A cell injection equipment (200) comprising the cell and tissue sheet forming package (100) of any of claims 1-10, comprising:
a carrier (50), including:
a base block (51), configured to carry the cell and tissue sheet forming package (100);
a top block (52), disposed pivotally on the base block (51) for positioning the cell and tissue sheet forming package (100) inside the base block (51), further having an injection hole (521); wherein, when the top block (52) is closed on the base block (51), a projection area of the injection hole (521) and another projection area of the hole (31) of the sliding door plate (30) of the cell and tissue sheet forming package (100) are overlapped;
at least one heating element (53), disposed at the base block (51) for providing thermal energy to the membrane (20) and the solution (SL); and
a positive magnetic assembly (54), disposed slidably between a third position and a fourth position at a top of the top block (52) in a manner of being parallel to the first direction (F1), configured to magnetically connect the passive magnetic assembly (32); wherein, when the positive magnetic assembly (54) slides to the fourth position, the sliding door plate (30) is slided synchronously to the second position;
an injection mechanism (60), including:
a fixing set (61), mounted on a rack (63) extending in perpendicular to the horizontal level surface;
an injection assembly (62), including:
a tube body (621), configured to contain the solution (SL), disposed at the fixing set (61) by being perpendicular to the horizontal level surface;
an injection head (622), coaxially disposed to a bottom of the tube body (621), having an injection tip (6221) protruding toward the horizontal level surface; and
a piston member (623), coaxially disposed in the tube body (621); and
a drive mechanism, including:
a first drive assembly (70), configured to drive the carrier (50) to undergo a reciprocating movement parallel to the horizontal level surface and the first direction (F1) between a fifth position and a sixth position; wherein, when the carrier (50) is at the fifth position, a projection area of the injection head (622) is deviated from another projection area of the injection hole (521) of the top block (52); wherein, when the carrier (50) is at the sixth position, the projection area of the injection head (622), the another projection area of the injection hole (521) of the top block (52), and a further projection area of the hole (31) of the sliding door plate (30) are overlapped;
a second drive assembly (80), configured to drive the fixing set (61) and the injection assembly (62) to undergo another reciprocating movement along the rack (63) perpendicular to the horizontal level surface between a seventh position and an eighth position; wherein, when the fixing set (61) and the injection assembly (62) are at the seventh position, a horizontal position of the injection tip (6221) is higher than that of a top surface of the top block (52) of the carrier (50); wherein, when the fixing set (61) and the injection assembly (62) are at the eighth position, the horizontal position of the injection tip (6221) is lower than that of the sealing film (40) on the hole (31) of the sliding door plate (30); and
a third drive assembly (90), configured to drive the piston member (623) to move perpendicular to the horizontal level surface inside the tube body (621) for pushing the solution (SL) out of the tube body (621) via the injection head (622).

12. The cell injection equipment (200) of claim 11, wherein a side of the carrier (50) is furnished with a first sensor (S1) and a second sensor (S2), and a first detection plate (D1) and a second detection plate (D2) are provided to the base block (51) of the carrier (50); wherein, when the first drive assembly (70) displaces the carrier (50), the first detection plate (D1) and the second detection plate (D2) are moved synchronously to approach corresponding detection ranges of the first sensor (S1) and the second sensor (S2); wherein, when the first sensor (S1) detects the first detection plate (D1), the first drive assembly (70) is stopped, and the carrier (50) is stopped at the fifth position; wherein, when the second sensor (S2) detects the second detection plate (D2), the first drive assembly (70) is stopped, and the carrier (50) is stopped at the sixth position.

13. The cell injection equipment (200) of claim 11, wherein a side of the injection mechanism (60) is furnished with a third sensor (S3), and a third detection plate (D3) is provided to the fixing set (61); wherein, when the second drive assembly (80) displaces the fixing set (61) and the injection assembly (62), the third detection plate (D3) is moved synchronously to approach a detection range of the third sensor (S3); wherein, when the third sensor (S3) detects the third detection plate (D3), the second drive assembly (80) is stopped, and the fixing set (61) is stopped at the seventh position.

14. The cell injection equipment (200) of claim 11, wherein the first drive assembly (70) includes:
a first screw bar (71), axially parallel to the horizontal level surface, mounting thereon the carrier (50); and
a first motor (72), configured to rotate the first screw bar (71) for further driving synchronously the carrier (50) to undergo a reciprocating movement between the fifth position and the sixth position.

15. The cell injection equipment (200) of claim 11, wherein the second drive assembly (80) includes:
a cam member (81), disposed under the fixing set (61);
a link member (82), having one end thereof to pivotally connect the fixing set (61) while another end thereof pivotally connects an eccentric point at the cam member (81); and
a drive member, configured to rotate the cam member (81) and further to displace synchronously the link member (82) and the fixing set (61) so as to have the fixing set (61) and the injection assembly (621) to undergo a reciprocating movement along the rack (63) between the seventh position and the eighth position in a manner perpendicular to the horizontal level surface.

16. The cell injection equipment (200) of claim 11, wherein the third drive assembly (90) includes:
a second screw bar (91), axially perpendicular to the horizontal level surface;
a depression member (92), disposed to the second screw bar (91), having one side thereof to face a top surface of the piston member (623) for the depression member (92) to depress the piston member (623) downward; and
a second motor (93), configured to rotate the second screw bar (91) so as further to displace synchronously the depression member (92) perpendicular to the horizontal level surface to perform a reciprocating movement between a ninth position and a tenth position.

17. The cell injection equipment (200) of claim 16, wherein one side of the cell injection equipment (200) is furnished with a fourth sensor (S4), and a fourth detection plate (D4) is provided to the depression member (92); wherein, when the third drive assembly (90) displaces the depression member (92), the fourth detection plate (D4) is moved synchronously to approach a detection range of the fourth sensor (S4); wherein, when the fourth sensor (S4) detects the fourth detection plate (D4), the third drive assembly (90) is stopped.

18. The cell injection equipment (200) of claim 11, wherein the passive magnetic assembly (32) has two passive magnetic elements (321) parallel to the horizontal level surface, and a line connecting the two passive magnetic elements (321) is perpendicular to the first direction (F1); wherein the positive magnetic assembly (54) includes two positive magnetic elements (541) for connecting magnetically the two passive magnetic elements (321).

## Patentansprüche

1. Verpackung (100) zur Bildung von Zell- und Gewebeschichten, umfassend:
einen Behälterkörper (10) mit einem Hohlraum (11);
eine in dem Hohlraum (11) angeordnete Membran (20) aus einem hydrophilen Material; und
eine Schiebetürplatte (30), die verschiebbar an der Oberseite des Behälterkörpers (10) parallel zu einer horizontalen Ebene und einer ersten Richtung (F1) angeordnet ist, wobei die Schiebetürplatte (30) zwischen einer ersten Position und einer zweiten Position an der Oberseite verschiebbar ist; wobei, wenn sich die Schiebetürplatte (30) in der ersten Position befindet, die Schiebetürplatte (30) für den Hohlraum (11) und die Membran (20) als Abdeckung dient; wobei, wenn sich die Schiebetürplatte (30) in der zweiten Position befindet, der Hohlraum (11) und die Membran (20) freigelegt sind;
**dadurch gekennzeichnet, dass** die Schiebetürplatte (30) umfasst:
ein Loch (31), das von einer Oberfläche (33) der Schiebetürplatte (30) zu einer Bodenfläche (34) der Schiebetürplatte (30) verläuft; wobei, wenn sich die Schiebetürplatte (30) in der ersten Position befindet, das Loch (31) über dem Hohlraum (11) und der Membran (20) für eine in dem Hohlraum angeordnete Lösung (SL) positioniert ist, wobei die Lösung (SL) eine Mischung aus einem biokompatiblen Polymermaterial und Zellen ist, die durch dieses Loch von der Oberfläche (33) der Schiebetürplatte (30) in den Hohlraum (11) injiziert werden soll; und
eine passive Magnetbaugruppe (32), die an der Schiebetürplatte (30) angeordnet ist, um sich mit einer positiven Magnetbaugruppe (54) magnetisch zu verbinden, wobei die positive Magnetbaugruppe (54) so konfiguriert ist, dass sie die Schiebetürplatte (30) verschiebt; und
einer Dichtungsfolie (40), die so konfiguriert ist, dass sie die Schiebetürplatte (30) und den Behälterkörper (10) abdeckt, um die Schiebetürplatte (30) und die Membran (20) abzudichten.

2. Verpackung (100) zur Bildung von Zell- und Gewebeschichten nach Anspruch 1, die ferner einen Begrenzungsrahmen (15) umfasst, der so konfiguriert ist, dass er fest in den Hohlraum (11) passt und somit auf einen Umfangsrand der Membran (20) drücken kann.

3. Verpackung (100) zur Bildung von Zell- und Gewebeschichten nach Anspruch 1, wobei die Tiefe des Hohlraums (11) größer ist als die Dicke der Membran (20).

4. Verpackung (100) zur Bildung von Zell- und Gewebeschichten nach Anspruch 1, wobei der Hohlraum (11) eine Tiefe H senkrecht zur horizontalen Ebene aufweist, die Membran (20) eine Dicke h senkrecht zur horizontalen Ebene aufweist, der Hohlraum (11) eine Fläche A parallel zur horizontalen Ebene aufweist und ein maximales Volumen der Lösung (SL) (H-h) x A beträgt.

5. Verpackung (100) zur Bildung von Zell- und Gewebeschichten nach Anspruch 1, wobei der Behälterkörper (10) einen flachen Bereich (13) aufweist, der Hohlraum (11) unterhalb des flachen Bereichs (13) angeordnet ist, zwei Gleitschienen (14) an gegenüberliegenden Seiten des flachen Bereichs (13) entsprechend dem Hohlraum (11) vorgesehen sind, die beiden Gleitschienen (14) sich einzeln in der ersten Richtung (F1) erstrecken und die Schiebetürplatte (30) zwischen den beiden Gleitschienen (14) angeordnet ist.

6. Verpackung (100) zur Bildung von Zell- und Gewebeschichten nach Anspruch 1, wobei der Hohlraum (11) ferner eine vorstehende Ecke (12) aufweist, die vorstehende Ecke (12) und die Aussparung (11) räumlich miteinander verbunden sind, und eine Kante der Membran (20) gegenüber der vorstehenden Ecke (12) exponiert ist, so dass zwischen der Membran (20) und der vorstehenden Ecke (12) ein Spalt entsteht.

7. Verpackung (100) zur Bildung von Zell- und Gewebeschichten nach Anspruch 1, wobei, wenn sich die Schiebetürplatte (30) in der ersten Position befindet, ein Vorsprungsbereich des Lochs (31) mit den Mittelpunkten des Hohlraums (11) und der Membran (20) übereinstimmt.

8. Verpackung (100) zur Bildung von Zell- und Gewebeschichten nach Anspruch 1, wobei die passive Magnetanordnung (32) zwei passive Magnetelemente (321) parallel zur horizontalen Ebene aufweist und eine Linie, die die beiden passiven Magnetelemente (321) verbindet, senkrecht zur ersten Richtung (F1) ist.

9. Verpackung (100) zur Bildung von Zell- und Gewebeschichten nach Anspruch 1, wobei das Polymermaterial eines von Kollagen, Gelatine, Hyaluronsäure, Alginat und PEG ist.

10. Verpackung (100) zur Bildung von Zell- und Gewebeschichten nach Anspruch 9, wobei die Zellen Fibroblasten, Myoblasten, Epithelzellen, Endothelzellen, Vorläuferzellen, Tenozyten, Stammzellen, mesenchymale Stammzellen, Knochenmarkstammzellen und Fettstammzellen sind.

11. Zellinjektionsvorrichtung (200), die die Verpackung (100) zur Bildung von Zell- und Gewebeschichten gemäß einem der Ansprüche 1 bis 10 umfasst, mit:
einem Träger (50), der umfasst:
einen Basisblock (51), der so konfiguriert ist, dass er die Verpackung (100) zur Bildung von Zell- und Gewebeschichten trägt;
einen oberen Block (52), der schwenkbar auf dem Basisblock (51) angeordnet ist, um die Verpackung (100) zur Bildung von Zell- und Gewebeschichten innerhalb des Basisblocks (51) zu positionieren, und der ferner eine Injektionsöffnung (521) aufweist; wobei, wenn der obere Block (52) auf dem Basisblock (51) geschlossen ist, ein Vorsprungsbereich der Injektionsöffnung (521) und ein weiterer Vorsprungsbereich der Öffnung (31) der Schiebetürplatte (30) der Verpackung (100) zur Bildung von Zell- und Gewebeschichten überlappen;
mindestens ein Heizelement (53), das am Basisblock (51) angeordnet ist, um der Membran (20) und der Lösung (SL) Wärmeenergie zuzuführen; und
eine positive Magnetbaugruppe (54), die verschiebbar zwischen einer dritten Position und einer vierten Position an einer Oberseite des oberen Blocks (52) parallel zur ersten Richtung (F1) angeordnet ist und so konfiguriert ist, dass sie die passive Magnetbaugruppe (32) magnetisch verbindet; wobei, wenn die positive Magnetbaugruppe (54) in die vierte Position gleitet, die Schiebetürplatte (30) synchron in die zweite Position gleitet;
ein Injektionsmechanismus (60), umfassend:
eine Fixiereinheit (61), die an einer sich senkrecht zur horizontalen Ebene erstreckenden Halterung (63) angebracht ist;
eine Injektionsbaugruppe (62), umfassend:
einen Rohrkörper (621), der so konfiguriert ist, dass er die Lösung (SL) enthält, und der senkrecht zur horizontalen Ebene an der Fixiereinheit (61) angeordnet ist;
einen Injektionskopf (622), der koaxial zu einem Boden des Rohrkörpers (621) angeordnet ist und eine Injektionsspitze (6221) aufweist, die in Richtung der horizontalen Ebene vorsteht; und
ein Kolbenelement (623), das koaxial im Rohrkörper (621) angeordnet ist; und
einen Antriebsmechanismus, der Folgendes umfasst:
eine erste Antriebsbaugruppe (70), die so konfiguriert ist, dass sie den Träger (50) antreibt, um eine Hin- und Herbewegung parallel zur horizontalen Ebene und zur ersten Richtung (F1) zwischen einer fünften Position und einer sechsten Position auszuführen; wobei, wenn sich der Träger (50) in der fünften Position befindet, ein Projektionsbereich des Injektionskopfes (622) von einem anderen Projektionsbereich des Injektionslochs (521) des oberen Blocks (52) abweicht; wobei, wenn sich der Träger (50) in der sechsten Position befindet, der Vorsprungsbereich des Injektionskopfes (622), der andere Vorsprungsbereich der Injektionsöffnung (521) des oberen Blocks (52) und ein weiterer Vorsprungsbereich der Öffnung (31) der Schiebetürplatte (30) überlappen;
eine zweite Antriebsbaugruppe (80), die so konfiguriert ist, dass sie die Fixiereinheit (61) und die Injektionsbaugruppe (62) antreibt, um eine weitere Hin- und Herbewegung entlang der Halterung (63) senkrecht zur horizontalen Ebene zwischen einer siebten Position und einer achten Position auszuführen; wobei, wenn sich die Fixiereinheit (61) und die Injektionsbaugruppe (62) in der siebten Position befinden, eine horizontale Position der Injektionsspitze (6221) höher ist als die der Oberfläche des oberen Blocks (52) des Trägers (50); wobei, wenn sich die Fixiereinheit (61) und die Injektionsvorrichtung (62) in der achten Position befinden, die horizontale Position der Injektionsspitze (6221) niedriger ist als die der Dichtungsfolie (40) auf dem Loch (31) der Schiebetürplatte (30); und
eine dritte Antriebsbaugruppe (90), die so konfiguriert ist, dass sie das Kolbenelement (623) antreibt, um sich senkrecht zur horizontalen Ebene innerhalb des Rohrkörpers (621) zu bewegen, um die Lösung (SL) über den Injektionskopf (622) aus dem Rohrkörper (621) herauszudrücken.

12. Zellinjektionsvorrichtung (200) nach Anspruch 11, wobei eine Seite des Trägers (50) mit einem ersten Sensor (S1) und einem zweiten Sensor (S2) versehen ist und eine erste Erfassungsplatte (D1) und eine zweite Erfassungsplatte (D2) an dem Basisblock (51) des Trägers (50) vorgesehen sind; wobei, wenn die erste Antriebsbaugruppe (70) den Träger (50) verschiebt, die erste Erfassungsplatte (D1) und die zweite Erfassungsplatte (D2) synchron bewegt werden, um sich entsprechenden Erfassungsbereichen des ersten Sensors (S1) und des zweiten Sensors (S2) zu nähern; wobei, wenn der erste Sensor (S1) die erste Erfassungsplatte (D1) erfasst, wird die erste Antriebsbaugruppe (70) angehalten und der Träger (50) wird an der fünften Position angehalten; wobei, wenn der zweite Sensor (S2) die zweite Erfassungsplatte (D2) erfasst, die erste Antriebsbaugruppe (70) angehalten wird und der Träger (50) an der sechsten Position angehalten wird.

13. Zellinjektionsvorrichtung (200) nach Anspruch 11, wobei eine Seite des Injektionsmechanismus (60) mit einem dritten Sensor (S3) versehen ist und eine dritte Detektionsplatte (D3) an der Fixiereinheit (61) vorgesehen ist; wobei, wenn die zweite Antriebsbaugruppe (80) die Fixiereinheit (61) und die Injektionsbaugruppe (62) verschiebt, die dritte Erfassungsplatte (D3) synchron bewegt wird, um sich einem Erfassungsbereich des dritten Sensors (S3) zu nähern; wobei, wenn der dritte Sensor (S3) die dritte Erfassungsplatte (D3) erfasst, die zweite Antriebsbaugruppe (80) angehalten wird und die Fixiereinheit (61) an der siebten Position angehalten wird.

14. Zellinjektionsvorrichtung (200) nach Anspruch 11, wobei die erste Antriebsbaugruppe (70) umfasst:
eine erste Schraubenstange (71), die axial parallel zur horizontalen Ebene verläuft und auf der der Träger (50) montiert ist; und
einen ersten Motor (72), der so konfiguriert ist, dass er die erste Schraubenstange (71) dreht, um den Träger (50) weiter synchron anzutreiben, damit er eine Hin- und Herbewegung zwischen der fünften Position und der sechsten Position ausführt.

15. Zellinjektionsvorrichtung (200) nach Anspruch 11, wobei die zweite Antriebsbaugruppe (80) umfasst:
ein Nocken (81), der unterhalb des Fixiereinheit (61) angeordnet ist;
ein Verbindungselement (82), dessen eines Ende schwenkbar mit der Fixiereinheit (61) verbunden ist, während sein anderes Ende schwenkbar mit einem Exzenterpunkt am Nocken (81) verbunden ist; und
ein Antriebselement, das so konfiguriert ist, dass es den Nocken (81) dreht und darüber hinaus das Verbindungselement (82) und die Fixiereinheit (61) synchron verschiebt, sodass die Fixiereinheit (61) und die Injektionsbaugruppe (621) eine Hin- und Herbewegung entlang der Halterung (63) zwischen der siebten Position und der achten Position in einer zur horizontalen Ebene senkrechten Weise ausführen.

16. Zelleninjektionsvorrichtung (200) nach Anspruch 1, wobei die dritte Antriebsbaugruppe (90) umfasst:
eine zweite Schraubenstange (91), die axial senkrecht zur horizontalen Ebene steht;
ein Druckelement (92), das an der zweiten Schraubenstange (91) angeordnet ist und mit einer Seite der Oberfläche des Kolbenelements (623) zugewandt ist, damit das Druckelement (92) das Kolbenelement (623) nach unten drückt; und
einen zweiten Motor (93), der so konfiguriert ist, dass er die zweite Schraubenstange (91) dreht, um das Druckelement (92) weiter synchron senkrecht zur horizontalen Ebene zu verschieben, um eine Hin- und Herbewegung zwischen einer neunten Position und einer zehnten Position auszuführen.

17. Zellinjektionsvorrichtung (200) nach Anspruch 16, wobei eine Seite der Zellinjektionsvorrichtung (200) mit einem vierten Sensor (S4) versehen ist und eine vierte Erfassungsplatte (D4) am Druckelement (92) vorgesehen ist; wobei, wenn die dritte Antriebsbaugruppe (90) das Druckelement (92) verschiebt, die vierte Erfassungsplatte (D4) synchron bewegt wird, um sich einem Erfassungsbereich des vierten Sensors (S4) zu nähern; wobei, wenn der vierte Sensor (S4) die vierte Erfassungsplatte (D4) erfasst, die dritte Antriebsbaugruppe (90) angehalten wird.

18. Zellinjektionsvorrichtung (200) nach Anspruch 11, wobei die passive Magnetbaugruppe (32) zwei passive Magnetelemente (321) parallel zur horizontalen Ebene aufweist und eine Linie, die die beiden passiven Magnetelemente (321) verbindet, senkrecht zur ersten Richtung (F1) verläuft; wobei die positive Magnetbaugruppe (54) zwei positive Magnetelemente (541) zum magnetischen Verbinden der beiden passiven Magnetelemente (321) umfasst.

## Revendications

1. Un emballage formant une feuille de cellules et de tissus(100), comprenant:
un corps de conteneur (10) comportant une cavité (11);
une membrane (20), disposée dans la cavité (11), réalisée en un matériau hydrophile; et
une plaque de porte coulissante (30), disposée de manière coulissante au sommet du corps de conteneur (10) de manière à être parallèle à une surface horizontale et à une première direction (F1), la plaque de porte coulissante (30) pouvant coulisser entre une première position et une deuxième position au sommet; dans lequel, lorsque la plaque de porte coulissante (30) se trouve dans la première position, la plaque de porte coulissante (30) recouvre la cavité (11) et la membrane (20); dans lequel, lorsque la plaque de porte coulissante (30) se trouve dans la deuxième position, la cavité (11) et la membrane (20) sont exposées;
**caractérisé en ce que** la plaque de porte coulissante (30) comprend:
un trou (31), pénétrant depuis une surface supérieure (33) de la plaque de porte coulissante (30) jusqu'à une surface inférieure (34) de la plaque de porte coulissante (30); dans lequel, lorsque la plaque de porte coulissante (30) est dans la première position, le trou (31) est positionné au-dessus de la cavité (11) et de la membrane (20) pour une solution (SL) disposée dans la cavité, dans laquelle la solution (SL) est un mélange d'un matériau polymère biocompatible et de cellules, à injecter à travers celle-ci dans la cavité (11) depuis la surface supérieure (33) de la plaque de porte coulissante (30); et
un ensemble magnétique passif (32), disposé au niveau de la plaque de porte coulissante (30) pour connecter magnétiquement un ensemble magnétique positif (54), l'ensemble magnétique positif (54) étant configuré pour faire coulisser la plaque de porte coulissante (30); et
un film d'étanchéité (40), configuré pour recouvrir la plaque de porte coulissante (30) et le corps de conteneur (10) de manière à sceller la plaque de porte coulissante (30) et la membrane (20).

2. Emballage formant une feuille de cellules et de tissus (100) selon la revendication 1, comprenant en outre un cadre de contrainte (15) configuré pour s'ajuster étroitement à la cavité (11) et ainsi pouvoir appuyer sur un rebord circonférentiel de la membrane (20).

3. Emballage pour la formation de feuilles de cellules et de tissus (100) selon la revendication 1, dans lequel la profondeur de la cavité (11) est supérieure à l'épaisseur de la membrane (20).

4. Emballage formant une feuille de cellules et de tissus (100) selon la revendication 1, dans lequel la cavité (11) a une profondeur H perpendiculaire à la surface horizontale, la membrane (20) a une épaisseur h perpendiculaire à la surface horizontale, la cavité (11) a une surface A parallèle à la surface horizontale, et le volume maximal de la solution (SL) est (H-h) x A.

5. Emballage formant une feuille de cellules et de tissus (100) selon la revendication 1, dans lequel le corps de conteneur (10) présente une zone peu profonde (13), la cavité (11) est disposée sous la zone peu profonde (13), deux glissières (14) sont prévues sur les côtés opposés de la zone peu profonde (13) en correspondance avec la cavité (11), les deux glissières (14) s'étendent individuellement dans la première direction (F1), et la plaque de porte coulissante (30) est disposée entre les deux glissières (14).

6. Emballage formant une feuille de cellules et de tissus (100) selon la revendication 1, dans lequel la cavité (11) comporte en outre un coin saillant (12), le coin saillant (12) et la cavité (11) sont reliés spatialement, et un bord de la membrane (20) est exposé au coin saillant (12) de manière à produire un espace entre la membrane (20) et le coin saillant (12).

7. Emballage formant une feuille de cellules et de tissus (100) selon la revendication 1, dans lequel, lorsque la plaque de porte coulissante (30) est dans la première position, une zone en saillie du trou (31) coïncide avec les centres de la cavité (11) et de la membrane (20).

8. Emballage formant une feuille de cellules et de tissus (100) selon la revendication 1, dans lequel l'ensemble magnétique passif (32) comporte deux éléments magnétiques passifs (321) parallèles à la surface horizontale, et une ligne reliant les deux éléments magnétiques passifs (321) est perpendiculaire à la première direction (F1).

9. Emballage formant une feuille de cellules et de tissus (100) selon la revendication 1, dans lequel le matériau polymère est l'un parmi le collagène, la gélatine, l'acide hyaluronique, l'alginate et le PEG.

10. Emballage formant une feuille de cellules et de tissus (100) selon la revendication 9, dans lequel les cellules sont l'une parmi les fibroblastes, les myoblastes, les cellules épithéliales, les cellules endothéliales, les cellules précurseurs, les ténocytes, les cellules souches, les cellules souches mésenchymateuses, les cellules souches de moelle osseuse et les cellules souches adipeuses.

11. Équipement d'injection de cellules (200) comprenant l'emballage formant une feuille de cellules et de tissus (100) de l'une quelconque des revendications 1 à 10, comprenant:
un support (50), comprenant :
un bloc de base (51), configuré pour transporter l'emballage formant une feuille de cellules et de tissus (100);
un bloc supérieur (52), disposé de manière pivotante sur le bloc de base (51) pour positionner l'emballage formant une feuille de cellules et de tissus (100) à l'intérieur du bloc de base (51), comportant en outre un trou d'injection (521); dans lequel, lorsque le bloc supérieur (52) est fermé sur le bloc de base (51), une zone en saillie du trou d'injection (521) et une autre zone en saillie du trou (31) de la plaque de porte coulissante (30) de l'emballage formant une feuille de cellules et de tissus (100) se chevauchent ;
au moins un élément chauffant (53), disposé au niveau du bloc de base (51) pour fournir de l'énergie thermique à la membrane (20) et à la solution (SL); et
un ensemble magnétique positif (54), disposé de manière coulissante entre une troisième position et une quatrième position au sommet du bloc supérieur (52) de manière à être parallèle à la première direction (F1), configuré pour connecter magnétiquement l'ensemble magnétique passif (32); dans lequel, lorsque l'ensemble magnétique positif (54) coulisse vers la quatrième position, la plaque de porte coulissante (30) coulisse de manière synchrone vers la deuxième position;
un mécanisme d'injection (60), comprenant:
un ensemble de fixation (61), monté sur un support (63) s'étendant perpendiculairement à la surface horizontale;
un ensemble d'injection (62), comprenant:
un corps de tube (621), configuré pour contenir la solution (SL), disposé au niveau de l'ensemble de fixation (61) de manière perpendiculaire à la surface horizontale;
une tête d'injection (622), disposée coaxialement à une partie inférieure du corps tubulaire (621), comportant une pointe d'injection (6221) faisant saillie vers la surface horizontale; et
un élément piston (623), disposé coaxialement dans le corps tubulaire (621); et
un mécanisme d'entraînement, comprenant:
un premier ensemble d'entraînement (70), configuré pour entraîner le support (50) à effectuer un mouvement de va-et-vient parallèle à la surface horizontale et à la première direction (F1) entre une cinquième position et une sixième position; dans lequel, lorsque le support (50) se trouve dans la cinquième position, une zone de projection de la tête d'injection (622) est déviée d'une autre zone de projection du trou d'injection (521) du bloc supérieur (52); dans lequel, lorsque le support (50) est dans la sixième position, la zone en saillie de la tête d'injection (622), l'autre zone en saillie du trou d'injection (521) du bloc supérieur (52) et une autre zone en saillie du trou (31) de la plaque de porte coulissante (30) se chevauchent;
un deuxième ensemble d'entraînement (80), configuré pour entraîner l'ensemble de fixation (61) et l'ensemble d'injection (62) à effectuer un autre mouvement de va-et-vient le long du support (63) perpendiculairement à la surface horizontale entre une septième position et une huitième position; dans lequel, lorsque l'ensemble de fixation (61) et l'ensemble d'injection (62) sont à la septième position, une position horizontale de la pointe d'injection (6221) est supérieure à celle de la surface supérieure du bloc supérieur (52) du support (50); dans lequel, lorsque l'ensemble de fixation (61) et l'ensemble d'injection (62) se trouvent dans la huitième position, la position horizontale de la pointe d'injection (6221) est inférieure à celle du film d'étanchéité (40) sur le trou (31) de la plaque de porte coulissante (30); et
un troisième ensemble d'entraînement (90), configuré pour entraîner l'élément de piston (623) à se déplacer perpendiculairement à la surface horizontale à l'intérieur du corps de tube (621) afin de pousser la solution (SL) hors du corps de tube (621) via la tête d'injection (622).

12. Équipement d'injection de cellules (200) selon la revendication 11, dans lequel un côté du support (50) est muni d'un premier capteur (S1) et d'un deuxième capteur (S2), et une première plaque de détection (D1) et une deuxième plaque de détection (D2) sont prévues sur le bloc de base (51) du support (50); dans lequel, lorsque le premier ensemble d'entraînement (70) déplace le support (50), la première plaque de détection (D1) et la deuxième plaque de détection (D2) sont déplacées de manière synchrone pour s'approcher des plages de détection correspondantes du premier capteur (S1) et du deuxième capteur (S2); dans lequel, lorsque le premier capteur (S1) détecte la première plaque de détection (D1), le premier ensemble d'entraînement (70) est arrêté et le support (50) est arrêté à la cinquième position; dans lequel, lorsque le deuxième capteur (S2) détecte la deuxième plaque de détection (D2), le premier ensemble d'entraînement (70) est arrêté et le support (50) est arrêté à la sixième position.

13. Équipement d'injection de cellules (200) selon la revendication 11, dans lequel un côté du mécanisme d'injection (60) est équipé d'un troisième capteur (S3), et une troisième plaque de détection (D3) est prévue sur l'ensemble de fixation (61); dans lequel, lorsque le deuxième ensemble d'entraînement (80) déplace l'ensemble de fixation (61) et l'ensemble d'injection (62), la troisième plaque de détection (D3) est déplacée de manière synchrone pour s'approcher d'une plage de détection du troisième capteur (S3); dans lequel, lorsque le troisième capteur (S3) détecte la troisième plaque de détection (D3), le deuxième ensemble d'entraînement (80) est arrêté et l'ensemble de fixation (61) est arrêté à la septième position.

14. Équipement d'injection de cellules (200) selon la revendication 11, dans lequel le premier ensemble d'entraînement (70) comprend:
une première barre à vis (71), parallèle axialement à la surface horizontale, sur laquelle est monté le support (50); et
un premier moteur (72), configuré pour faire tourner la première barre à vis (71) afin d'entraîner de manière synchrone le support (50) pour qu'il effectue un mouvement de va-et-vient entre la cinquième position et la sixième position.

15. Équipement d'injection de cellules (200) selon la revendication 11, dans lequel le deuxième ensemble d'entraînement (80) comprend:
un élément de came (81), disposé sous l'ensemble de fixation (61);
un élément de liaison (82), dont une extrémité est reliée de manière pivotante à l'ensemble de fixation (61) tandis que l'autre extrémité est reliée de manière pivotante à un point excentrique de l'élément de came (81); et
un élément d'entraînement, configuré pour faire tourner l'élément de came (81) et pour déplacer de manière synchrone l'élément de liaison (82) et l'ensemble de fixation (61) de manière à ce que l'ensemble de fixation (61) et l'ensemble d'injection (621) subissent un mouvement alternatif le long du support (63) entre la septième position et la huitième position d'une manière perpendiculaire à la surface horizontale.

16. Équipement d'injection de cellules (200) selon la revendication 1, dans lequel le troisième ensemble d'entraînement (90) comprend:
une deuxième barre à vis (91), perpendiculaire axialement à la surface horizontale;
un élément de dépression (92), disposé sur la deuxième barre à vis (91), dont un côté est tourné vers une surface supérieure de l'élément de piston (623) afin que l'élément de dépression (92) puisse appuyer sur l'élément de piston (623) vers le bas; et
un deuxième moteur (93), configuré pour faire tourner la deuxième barre à vis (91) de manière à déplacer davantage de manière synchrone l'élément de dépression (92) perpendiculairement à la surface horizontale afin d'effectuer un mouvement de va-et-vient entre une neuvième position et une dixième position.

17. Équipement d'injection de cellules (200) selon la revendication 16, dans lequel un côté de l'équipement d'injection de cellules (200) est muni d'un quatrième capteur (S4), et une quatrième plaque de détection (D4) est prévue sur l'élément de pression (92) ; dans lequel, lorsque le troisième ensemble d'entraînement (90) déplace l'élément de dépression (92), la quatrième plaque de détection (D4) est déplacée de manière synchrone pour s'approcher d'une plage de détection du quatrième capteur (S4); dans lequel, lorsque le quatrième capteur (S4) détecte la quatrième plaque de détection (D4), le troisième ensemble d'entraînement (90) est arrêté.

18. Équipement d'injection de cellules (200) selon la revendication 11, dans lequel l'ensemble magnétique passif (32) comporte deux éléments magnétiques passifs (321) parallèles à la surface horizontale, et une ligne reliant les deux éléments magnétiques passifs (321) est perpendiculaire à la première direction (F1); dans lequel l'ensemble magnétique positif (54) comprend deux éléments magnétiques positifs (541) pour relier magnétiquement les deux éléments magnétiques passifs (321).
